# EUROPÄISCHE PATENTSCHRIFT

(11) **EP 2 153 800 B1**
(45) Veröffentlichungstag und Bekanntmachung des Hinweises auf die Patenterteilung: **07.06.2017**
(21) Anmeldenummer: 09009917.7
(22) Anmeldetag: 31.07.2009
(51) Int. Cl.: B21F 27/00, B21F 99/00, A61F 2/00, B21F 45/00, A61F 2/30, B22F 7/00

(54) **Künstliches Gelenk**
Artificial joint
Articulation artificielle

(30) Priorität: 11.08.2008 DE 102008037201
(43) Veröffentlichungstag der Anmeldung: 17.02.2010
(73) Patentinhaber: Buck, Alfred Ernst, 71149 Bondorf (DE)
(72) Erfinder: Buck, Alfred Ernst, 71149 Bondorf (DE)
(74) Vertreter: Herden, Andreas F.

(56) Entgegenhaltungen:
- EP-A1- 0 235 606
- EP-A1- 0 526 682
- EP-A2- 0 178 650
- EP-A2- 1 629 796
- WO-A1-2008/088869
- WO-A2-2005/114120
- AU-A1- 2006 201 728
- FR-A1- 2 664 501
- GB-A- 2 162 065
- JP-A- 8 140 996
- US-A- 3 867 728
- US-A- 4 570 271
- US-A- 4 997 445
- US-A1- 2002 123 750
- US-A1- 2005 146 070

## Beschreibung

Die vorliegende Erfindung betrifft ein Implantat für den chirurgischen Einsatz bei Menschen oder Wirbeltieren zum Ersatz, zum Teilersatz oder zum Verstärken eines Gelenks oder einer Bandscheibe und ein Verfahren zur Herstellung eines Implantats.

### Hintergrund der Erfindung

Gelenke stellen die Verbindungsstellen zwischen den Knochen dar und sind für die Beweglichkeit des Körpers zuständig. Neben dem Gewährleisten der Beweglichkeit leisten die Gelenke dabei auch das Auffangen bzw. Kompensieren des beim Stehen und Aufspringen entstehenden Drucks auf das Skelettsystem.

Aufgrund von Erkrankungen und/oder Verschleiß kann es erforderlich sein, die natürlichen Gelenke durch künstliche Gelenke, die als Implantate in die Knochen eingesetzt werden, auszutauschen. Beispielsweise werden bei einem Gelenk, wie einem Schulter-, Hüft-, oder Kniegelenk, Gelenkkugel bzw. Gelenkkopf und Gelenkpfanne mit Implantaten aus Kunststoff getauscht, welche fest im Knochen verankert werden müssen. Es wird in diesem Zusammenhang unterschieden zwischen der Totalendoprothese, bei der sowohl Gelenkkopf als auch Gelenkpfanne ersetzt werden, und der der Teilendoprothese, bei der nur der Gelenkkopf ausgetauscht wird.

Die Verankerung der Implantate, zum Beispiel mit einem Knochen, erfolgt im Allgemeinen durch ein Einwachsen von Knochen bzw. Gewebe und/oder durch ein Einzementieren. Zum Teil kann die Verankerung auch über ein Verschrauben erfolgen. Zum Beispiel werden Hüftpfannen in der Regel in den Knochen eingeschraubt.

Das Implantat ist im Allgemeinen an die Eigenschaften des zu ersetzenden Gelenks, wie Abmessungen, Form und die statischen und/oder dynamischen Eigenschaften, angepasst, um eine im Rahmen der natürlichen Bewegung uneingeschränkte Bewegung zu ermöglichen. Die dynamischen Eigenschaften, beziehen sich hierbei insbesondere auf die Steifigkeit, Elastizität, Federung und/oder Dämpfung des Implantats.

In der DE 10 2004 041 354 A1 ist ein Implantat beschrieben, in welchem der federnde oder elastische Teil eines Gelenks oder einer Bandscheibe durch eine gepresste Maschenware bereitgestellt wird. Die Maschenware selbst wird durch einen Draht bereitgestellt. Der Inhalt der genannten Anmeldung, insbesondere das beschriebene Implantat und das Verfahren zur Herstellung des Implantats, wird in vollen Umfang in die vorliegende Anmeldung inkorporiert. Die Porösität der gepressten Maschenware kann ein festes Verwachsen des Implantats mit dem Knochen bzw. dem Gewebe durch Knochen- bzw. Gewebeeinwuchs in das Implantat ermöglichen.

Sofern jedoch der dort beschriebene Draht durch ein sogenanntes bioinertes Material, wie zum Beispiel Titan, gebildet wird, kann die Möglichkeit bestehen, dass der Knochen bzw. das Gewebe soweit oder sogar vollständig in das Implantat einwächst, dass das Implantat die Anforderungen, insbesondere bezüglich seiner dynamischen Eigenschaften, nicht mehr erfüllen kann. Es kann hierbei sogar zu einer unerwünschten Versteifung des Implantats führen.

Zudem werden auch an die Oberflächen des Implantats, die zueinander bewegt werden, hohe Anforderungen hinsichtlich einer guten Gleitfähigkeit bei minimaler Reibung gestellt. Es sollte auch im Wesentlichen kein Abrieb erzeugt werden.

Das Dokument US 2005/146070 A1 zeigt ein Implantat, bei welchem ein Kunststoffkörper mit einem metallischen Netz verbunden ist.

### Allgemeine Beschreibung der Erfindung

Vor diesem Hintergrund hat sich die vorliegende Erfindung daher zur Aufgabe gestellt, ein Implantat und ein Verfahren zur Herstellung eines Implantats bereitzustellen, welche die vorstehend beschriebenen Nachteile des Standes der Technik zumindest vermindern.

Hierbei soll es insbesondere möglich sein, bereits bestehende Implantate derart zu erweitern, dass sowohl eine verbesserte Verankerung des Implantats im Gewebe bzw. im Knochen als auch eine verbesserte Funktionalität des Gelenks bereitgestellt werden.

Gelöst werden diese Aufgaben durch die Implantate und die Verfahren gemäß der unabhängigen Ansprüche. Vorteilhafte Ausführungsformen sind Gegenstand der jeweiligen Unteransprüche.

Die Erfindung betrifft ein Implantat für den chirurgischen Einsatz bei Menschen oder Wirbeltieren zum Ersatz, zum Teilersatz oder zum Verstärken eines Gelenks umfassend einen Verbund aus
- einem ersten Abschnitt, der durch einen gepressten Formkörper aus Draht gebildet ist, und
- einem zweiten Abschnitt, wobei der Formkörper abschnittsweise in den zweiten Abschnitt eingebettet ist.

Das erfindungsgemäße Implantat kann durch ein Verfahren mit folgenden Schritten hergestellt werden:
- Bereitstellen einer Maschenware aus Draht,
- Falten und/oder Aufrollen der Maschenware,
- Pressen der Maschenware zu einem Formkörper zum Ausbilden eines ersten Abschnitts,
- Bereitstellen eines zweiten Abschnitts und
- Verbinden von dem ersten Abschnitt und dem zweiten Abschnitt, wobei der Formkörper teilweise in dem zweiten Abschnitt eingebettet wird.

Der erste Abschnitt und der zweite Abschnitt können auch als erster bzw. zweiter Körper bezeichnet werden.

Ein gepresster Formkörper ist ein Körper, der durch einen Pressvorgang in eine zu erzielende Form überführt wird. Die Gestalt des Formkörpers wird im Wesentlichen durch den Preßvorgang gebildet. Der Formkörper stellt im Wesentlichen die dynamischen Eigenschaften des Implantats bereit. Für Details zur Herstellung des Formkörpers sei auf die später folgenden Ausführungen verwiesen.

Der gepresste Formkörper ist, da er durch Draht gebildet wird oder aus Draht besteht, ein poröser Körper. Diese poröse Struktur ermöglicht, dass der Formkörper oder der Draht des Formkörpers zumindest teilweise oder teilweise in den zweiten Abschnitt eingebettet sein kann. Unter einem Einbetten wird verstanden, dass zum einen die Drähte des Formkörpers in das Volumen oder wenigstens der Oberfläche des zweiten Abschnitts eingedrungen und dort fixiert sind. Durch das Einbetten wird ein Teil der Hohlräume des porösen Formkörpers durch das Material des zweiten Abschnitts gefüllt oder teilweise gefüllt. Um einen stabilen Verbund herstellen und die dynamischen Eigenschaften bereitstellen zu können, ist die Seite des Formkörpers bis zur Hälfte, bevorzugt bis zu einem Drittel, besonders bevorzugt von bis zu einem Fünftel seiner Dicke in dem zweiten Abschnitt eingebettet. In einer Variante der Erfindung sind der erste Abschnitt, vorzugsweise der Formkörper, und der zweite Abschnitt stoffschlüssig miteinander verbunden.

Der zweite Abschnitt selbst ist vorzugsweise kein gepresster Formkörper aus Draht. Er besitzt andere Eigenschaften als der erste Abschnitt oder der Formkörper. Der zweite Abschnitt besitzt eine gegenüber dem Formkörper reduzierte Porösität. Der zweite Abschnitt ist vorzugsweise massiv bzw. nicht porös. In einer Ausführungsform wird der zweite Abschnitt durch ein nichtmetallisches Material gebildet.

Der zweite Abschnitt ist oder umfasst wenigstens ein Material aus einer Gruppe bestehend aus Keramik und Kunststoff. Vorzugsweise wird der zweite Abschnitt, insbesondere zunächst, zumindest abschnittsweise mit einer derart niedrigen Viskosität bereitgestellt, die ein Einbetten oder Aufnehmen des ersten Abschnitts, vorzugsweise des Formkörpers, ermöglicht. Dies kann im Allgemeinen durch ein Erwärmen des Materials des zweiten Abschnitts erreicht werden, so dass das Material verformbar oder zum Beispiel sogar "flüssig" wird. Durch ein Verfestigen des zweiten Abschnitts wird der stabile Verbund hergestellt.

In einer ersten Ausführungsform kann der zweite Abschnitt zum einen direkt als Körper bereitgestellt und dann mit dem ersten Abschnitt verbunden werden. In einer weiteren Ausführungsform ist oder wird der zweite Abschnitt erst auf dem ersten Abschnitt, vorzugsweise dem Formkörper, ausgebildet. Zum Beispiel im Fall der Ausbildung des Implantats durch einen Kunststoff ist oder wird das Material zur Ausbildung des zweiten Abschnitts auf dem ersten Abschnitt, vorzugsweise dem Formkörper, aufgespritzt. Zum Beispiel im Fall der Ausbildung des Implantats durch eine Keramik ist oder wird das Material zur Ausbildung des zweiten Abschnitts auf dem ersten Abschnitt, vorzugsweise dem Formkörper, aufgesintert.

Zur Ausbildung einer Gelenkpfanne oder eines Gelenkkopfs ist der erste Abschnitt, vorzugsweise der Formkörper, als eine Art erste Schale mit einer Innenseite und einer Außenseite ausgebildet. Der zweite Abschnitt istan der Innenseite des ersten Abschnitts angeordnet. Vorzugsweise ist der zweite Abschnitt auch als eine Art zweite Schale mit einer Innenseite und einer Außenseite ausgebildet. Die erste Schale und die zweite Schale liegen in einer Version ineinander oder aneinander. Erfindungsgemäß bildet der Innenraum der zweiten Schale den Aufnahmebereich für den Gelenkkopf. Der Gelenkkopf gleitet dabei an der Innenseite der zweiten Schale.

Der zweite Abschnitt stellt im Wesentlichen die gleitenden Eigenschaften des Implantats bereit. Er trägt zu den dynamischen Eigenschaften des Implantats bei. Der zweite Abschnitt ist an einer Seite des Implantats angeordnet, welche eine Gleitfläche für eine Bewegung in einem Gelenk bildet. Der zweite Abschnitt bildet somit die oder eine Gleitfläche des Implantats. Bei einem Gelenkkopf ist dies die Oberseite oder Außenseite des Gelenkkopfs. Bei einer Gelenkpfanne ist dies die Unterseite oder Außenseite der Gelenkpfanne.

Der erste Abschnitt, vorzugsweise der Formkörper, ist dagegen an einer Seite des Implantats angeordnet, die an das umliegende Gewebe oder an den anliegenden Knochen grenzt, an das bzw. dem die Befestigung oder Verankerung des Implantats erfolgen soll. Der erste Abschnitt, vorzugsweise der Formkörper, bildet den Befestigungsteil. Bei einem Gelenkkopf ist dies die Unterseite oder Innenseite des Gelenkkopfs. Bei einer Gelenkpfanne ist dies die Oberseite oder Innenseite der Gelenkpfanne.

In einer Ausführungsform der Erfindung ist der gepresste Formkörper, vorzugsweise der Draht des gepressten Formkörpers, zumindest abschnittsweise beschichtet. Im Detail ist der Draht des gepressten Formkörpers aus einem ersten Material gebildet ist, wobei der Draht des Formkörpers zumindest abschnittsweise oder abschnittsweise eine Beschichtung aus einem zweiten Material aufweist.

Vorzugsweise beinhaltet dies das Ausbilden einer Beschichtung, insbesondere einer teilweisen Beschichtung, auf dem Draht mit einem zweiten Material, wobei das erste Material oder das zweite Material durch ein Material bereitgestellt wird, welches, in einem eingesetzten Zustand des Implantats, an einem Grenzbereich zu einem umgebenden Gewebe oder Knochen zumindest abschnittsweise ein Einwachsen oder Ausbilden des Gewebes wenigstens in einen Oberflächenbereich des Formkörpers ermöglicht.

Bestimmte Materialien, zum Beispiel Titan, neigen dazu, wie bereits in der Einleitung ausgeführt, in das Gewebe, wie Knochen und/oder Knorpel einzuwachsen bzw. ermöglichen ein Einwachsen des Gewebes in ein Implantat bzw. einen porösen Körper. Dies kann unter Umständen dazu führen, dass ein Implantat seine dynamischen Eigenschaften und damit seine Funktion verliert. Dieser vermeintliche Nachteil kann jedoch als Vorteil genutzt werden, da sich eine äußerst feste Verbindung zwischen dem Implantat und dem Gewebe des Körpers ausbildet.

Durch eine gezielte Auswahl der Stellen des Implantats oder des Formkörpers, an denen ein Einwachsen oder Verbinden ermöglicht werden soll, kann zum einen eine feste Verbindung zu dem benachbarten oder angrenzenden Gewebe hergestellt werden und zum anderen gleichzeitig die dynamischen Eigenschaften des Implantats erhalten werden.

Die Auswahl, ob das erste Material oder das zweite Material mit dem "verbindenden" Material bereitgestellt wird, ist abhängig davon, welches Material den Randbereich zu dem umliegenden Gewebe oder Knochen bildet, so dass ein stabiler Verbund mit dem Gewebe oder Knochen ausgebildet werden kann.

Vorzugsweise soll das Einwachsen oder das Verbinden in dem Grenzbereich zwischen Gewebe und Implantat ermöglicht werden. Bei einem Implantat in der Ausgestaltung einer Bandscheibe ist bzw. sind dies die Oberseite und/oder die Unterseite der Bandscheibe. Hierbei ist der Formkörper nierenförmig mit einer gebogenen konvexen Seite und einer gegenüberliegenden konkaven Seite ausgebildet. Bei einem Implantat in der Ausgestaltung einer Gelenkpfanne oder eines Gelenkkopfes ist dies die Rückseite der Gelenkpfanne bzw. des Gelenkkopfes. Hierbei kann der Formkörper als eine Art Schale ausgebildet sein.

In einer Ausführungsform der Erfindung wird der Draht vor dem Ausbilden des Formkörpers an den Stellen, die nach dem Ausbilden des Formkörpers den gewünschten Abschnitt des Implantats bilden, entsprechend ihrer Funktion bearbeitet.

Fördert zum Beispiel das ausgewählte Material des Drahtes, wie Titan und/oder seine Legierungen, ein Einwachsen des Gewebes, so wird der Draht abschnittsweise oder vollständig mit einer Beschichtung versehen, die ein Einwachsen im Wesentlichen reduziert oder unterdrückt. Ein Beispiel für ein solches Material stellt ein Kunststoff, wie zum Beispiel Silikon und/oder Teflon, und/oder ein Edelmetall, wie zum Beispiel Gold, dar.

Wird jedoch durch das ausgewählte Material des Drahtes ein Einwachsen des Gewebes unterdrückt, so wird der Draht abschnittsweise oder vollständig mit einer Beschichtung versehen, die das Einwachsen im Wesentlichen fördert oder ermöglicht. Ein Beispiel für ein solches Material stellt, wie bereits vorstehend ausgeführt, Titan und/oder seine Legierungen dar.

In einer bevorzugten Ausführungsform der Erfindung wird der Draht, insbesondere der Draht an sich oder der beschichtete Draht, erst nach dem Ausbilden des Formkörpers an den Stellen, die nach dem Ausbilden des Formkörpers den gewünschten Abschnitt des Implantats bilden, entsprechend ihrer Funktion bearbeitet. Vorzugsweise soll das Einwachsen in einem Grenzbereich zwischen Gewebe und Implantat zumindest abschnittsweise ermöglicht werden.

Ist der Draht mit einer Beschichtung versehen, die kein Einwachsen ermöglicht, so kann der Grenzbereich des Implantats derart bearbeitet werden, dass wenigstens in einem Oberflächenbereich ein Einwachsen ermöglicht wird. Als eine Bearbeitung bietet sich ein Entfernen der Beschichtung an. In einer Ausführungsform erfolgt das Entfernen durch ein Ätzen. Hierbei bietet sich ein einfaches Eintauchen des Implantats an. Über die Eintauchtiefe und/oder die Eintauchdauer wird bzw. werden die Eigenschaften in dem Grenzbereich eingestellt. Somit besitzt der Formkörper in einer Ausführungsform keine Beschichtung an wenigstens einer seiner Seiten, welche, im eingesetzten Zustand des Implantats, an ein umgebendes Gewebe grenzt, so dass ein Einwachsen des Gewebes, insbesondere in einen Oberflächenbereich, des Formkörpers ermöglicht wird. Dazu wird nach dem Pressen des Formkörpers an der zumindest einen Seite des Formkörpers die Beschichtung entfernt, so dass die Oberfläche des Drahts freigelegt wird.

Wird der Draht mit einem Material bereitgestellt, welches kein Einwachsen ermöglicht, so kann der Grenzbereich des Implantats derart bearbeitet werden, dass wenigstens in einem Oberflächenbereich ein Einwachsen ermöglicht wird. Als eine Bearbeitung bietet sich das Aufbringen einer Beschichtung auf dem Draht an. In einer Ausführungsform erfolgt ein Galvanisieren des Drahtes. Hierbei bietet sich ein einfaches Eintauchen des Implantats an. Über die Eintauchtiefe und/oder die Eintauchdauer wird bzw. werden die Eigenschaften in dem Grenzbereich eingestellt. Somit ist in einer weiteren Ausführungsform das Implantat im eingesetzten Zustand an einem Grenzbereich zu einem umgebenden Gewebe beschichtet, so dass ein Einwachsen des Gewebes, vorzugsweise wenigstens in einem Oberflächenbereich, ermöglicht wird. Diese Beschichtung an der zumindest einen Seite des Formkörpers wird nach dem Pressen ausgebildet.

Um ein zu tiefes Einwachsen des Gewebes zu unterbinden, verbleiben die Drähte des Formkörpers, welche des Kern des Formkörpers bilden, mit einem Material oder einer Beschichtung, welches bzw. welche das Einwachsen des Gewebes in den Kern des Formkörpers unterdrückt.

Das erste Material oder das zweite Material ist oder umfasst ein im Wesentlichen bioinertes und/oder bioaktives Material. Das erste Material und das zweite Material besitzen unterschiedliche Eigenschaften.

Boinerte Materialien sind zum Beispiel Titan, Aluminium und/oder Zirkonium. Ohne an diese Theorie gebunden zu sein, wird davon ausgegangen, dass es zu einer Kontaktosteogenese kommt. Ein bioinertes Material wird dabei von dem Gewebe oder einem fibrösen Bindegewebe umgeben. Es scheint eine Art morphologische Fixierung zu sein, bei der sich nicht poröse inerte Materialien mit dem umgebenden Gewebe durch das Einwachsen des Knochens in die oberflächlichen Unebenheiten verbinden. Die Gewebefreundlichkeit von Titan oder seiner Legierungen beruht wohl auf einer vorhandenen Oxidschicht. Diese trennt das Implantat vom dem umgebenden Gewebe oder Bindegewebe. Es wird somit eine direkte Knochenbindung ermöglicht. Das gleiche scheint auch für Aluminium bzw. Aluminiumoxid und/oder Zirkonium bzw. Zirkoniumoxid zu gelten.

Bioaktive Materialien sind zum Beispiel Glaskeramiken und/oder Hydroxylapatite. Ohne an diese Theorie gebunden zu sein, wird davon ausgegangen, dass eine Verbundosteogenese gebildet wird. Bioaktive Materialen gehen eine direkte Verbindung mit dem Umgebungsgewebe ein. Es ist wohl eine physikalisch-chemische Verbindung mit dem Knochen, wodurch ein stabiler Verbund gebildet wird.

Die folgenden Ausführungen beziehen sich im Wesentlichen auf den gepressten Formkörper an sich. Dieser wird durch einen Draht gebildet. Je nach Anforderung besteht der Draht aus oder umfasst der Draht im Allgemeinen Titan, eine Titanlegierung, Edelstahl oder eine Edelstahllegierung. Der Draht besitzt einen Durchmesser von etwa 0,01 mm bis 5 mm, bevorzugt von etwa 0,05 mm bis etwa 1 mm, besonders bevorzugt von etwa 0,2 mm bis etwa 0,3 mm.

In einer Ausgestaltung der Erfindung ist oder wird der gepresste Formkörper aus einer Maschenware ausgebildet. Um eine regelmäßige Struktur zu erhalten, ist oder wird die Maschenware vorzugsweise als ein Gestrick ausgebildet. In einer bevorzugten Ausgestaltung ist oder wird das Gestrick als ein Rundgestrick ausgebildet. Dadurch können Kanten und das Auftreten von möglichen Inhomogenitäten in dem Formkörper wirksam unterbunden werden. Die Maschenware kann zudem noch mit einer Prägung versehen werden.

Der gepresste Formkörper ist oder wird insbesondere aus einer gefalteten und/oder aufgerollten Maschenware ausgebildet. Durch das Falten und/oder Rollen kann bzw. können die dynamischen Eigenschaften, insbesondere über die Dichte der Maschenware, eingestellt werden. Vorzugsweise wird die Maschenware zunächst gefaltet und dann aufgerollt. Der Pressvorgang bzw. der formgebende Schritt zur Ausbildung des Formkörpers erfolgt mit einem hydraulischen oder pneumatischen Presswerkzeug in einer der Gestalt des Formkörpers entsprechenden Negativform.

Der gebildete Formkörper weist einen Porositätsgrad zwischen 20% und 80%, bevorzugt zwischen 30% und 70%, besonders bevorzugt zwischen 20% und 60% auf. Der Formkörper besitzt eine Maschenweite von 0,01 mm bis 50 mm, bevorzugt von 0,5 mm bis 20 mm, besonders bevorzugt von 3 mm bis 8 mm. Das Implantat oder der Formkörper ist zumindest abschnittsweise elastisch. Es bzw. er besitzt gemäß der Erfindung eine Federkonstante, die, insbesondere anfänglich, zwischen 50 und 3000 N/mm, bevorzugt zwischen 100 und 1000 N/mm, besonders bevorzugt-zwischen 150 und 800 N/mm beträgt.

Die vorliegende Erfindung wird anhand der nachfolgenden Ausführungsbeispiele im Einzelnen erläutert. Hierzu wird auf die beiliegenden Zeichnungen Bezug genommen. Die gleichen Bezugszeichen in den einzelnen Zeichnungen beziehen sich auf die gleichen Teile.
Figur 1 zeigt schematisch einen Aufbau einer Endoprothese für ein Hüftgelenk.
Figuren 2.a und 2.b zeigen schematisch die Hüftpfanne aus Figur 1 in einem Querschnitt und einer perspektivischen Ansicht.
Figuren 3.a und 3.b zeigen schematisch jeweils einen Querschnitt eines erfindungsgemäßen Hüftpfannenimplantats.
Figuren 4.a bis 4.c zeigen eine detaillierte Darstellung eines Formkörpers für eine Hüftpfanne (ohne den zweiten Abschnitt) in einer Seitenansicht, einer Unteransicht und einer perspektivischen Ansicht.
Figuren 5.a und 5.b illustrieren einen Ausschnitt einer Wirbelsäule und ein Bandscheibenimplantat, welches nicht Gegenstand der Erfindung ist.
Figuren 6.a und 6.b zeigen schematisch jeweils einen Querschnitt eines nicht erfindungsgemäßen Bandscheibenimplantats.
Figur 7 zeigt eine Detailansicht eines nicht erfindungsgemäßen Bandscheibenimplantats.

### Detaillierte Beschreibung der Erfindung

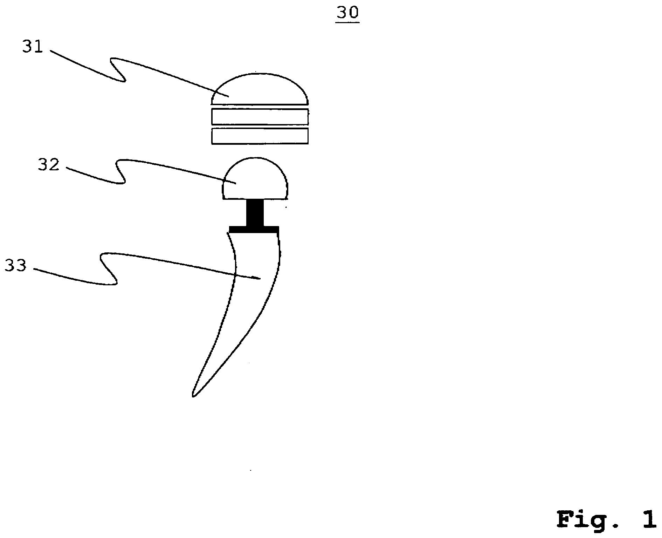

Figur 1 zeigt schematisch einen Aufbau einer Endoprothese für ein Hüftgelenk 30. Es ist eine Totalendoprothese dargestellt, bei der sowohl die Gelenkpfanne 31 als auch der Gelenkkopf 32 ersetzt werden. Der Gelenkkopf 32 kann im Knochen zum Beispiel über einen Schaft 33 fest verankert werden. Das Hüftgelenk 30 ist eine Art Kugelgelenk. Das kugelige Ende des einen Teils 32 greift in die hohlkugelförmige Pfanne des anderen Teils 31 ein, wodurch vorzugsweise ein Schwingen nach allen Seiten möglich wird. Die Erfindung wird beispielhaft in der Anwendung in einem Hüftgelenk 30 dargestellt. Die Anwendung in einem anderen Gelenk, wie zum Beispiel Schulter- Hüft- und/oder Kniegelenk, ist ebenso möglich.

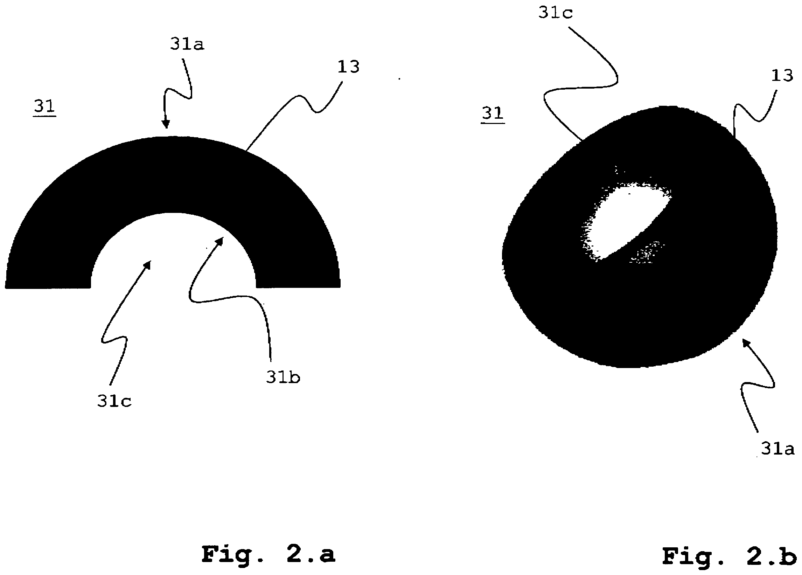

Figur 2.a zeigt schematisch die Hüftpfanne 31 aus Figur 1 in einem Querschnitt. Bekannte Hüftpfannen 31 bestehen oft aus Spezialkunststoffen oder Keramiken. Die Hüftpfanne 31 ist als eine Art Schale 13 oder Halbkugelschale 13 ausgebildet. Eine Halbkugelschale ist die Differenzmenge zweier im Wesentlichen konzentrischer Halbkugeln mit unterschiedlichem Radius. Dies ist noch einmal in Figur 2.b in einer perspektivischen Darstellung illustriert. Die Hüftpfanne 31 wird über ihre Außenseite 31a mit dem Knochen 50 oder dem Gewebe 50 verbunden. Der Innenraum 31c der Hüftpfanne 31 nimmt den Gelenkkopf 32 zumindest abschnittsweise auf. Die Bewegung des Gelenkkopfs 32 wird mit der Hüftpfanne 31 sozusagen geführt. Die Innenseite 31a der Hüftpfanne 31 und die Außenseite des nicht dargestellten Gelenkkopfs 32 liegen dabei aneinander. Diese sind im Allgemeinen jeweils derart glatt, dass eine gleitende Bewegung bei minimaler Reibung, vorzugsweise ohne wesentlichen Abrieb, erzielt werden soll.

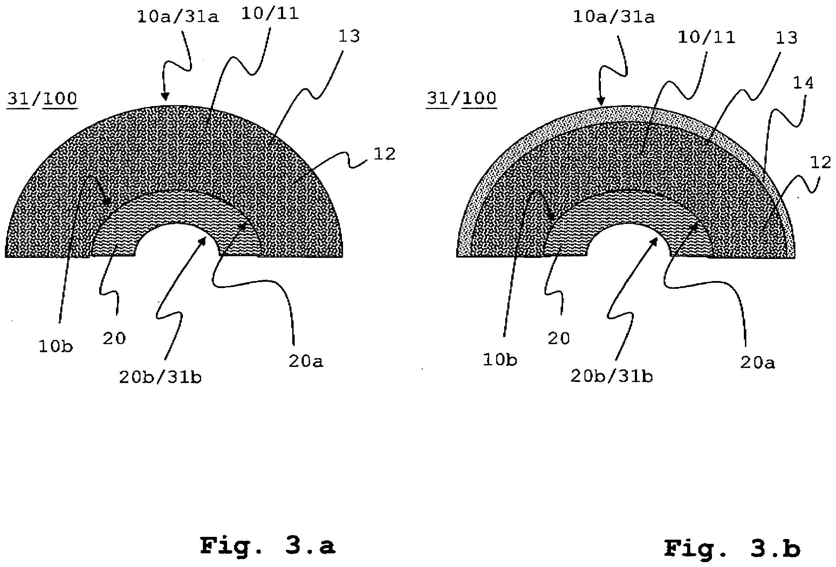

Figur 3.a zeigt schematisch einen Querschnitt eines erfindungsgemäßen Implantats 100 für eine Hüftpfanne 31. Das Implantat 100 ist aus mehreren Abschnitten oder mehreren Teilen aufgebaut. Das Implantat 100 bzw. die Hüftpfanne umfasst oder besteht vorliegend aus einem gepressten Formkörper 11 aus Draht 12, insbesondere aus einem Formkörper 11 aus einer gepressten Maschenware aus Draht 11, als einem ersten Abschnitt 10 und aus einem Kunststoffkörper als zweitem Abschnitt 20.

Der zweite Abschnitt 20 ist im Innenraum des ersten Abschnitts 10 bzw. des Formkörpers 11 angeordnet. Der Formkörper 11 umgreift sozusagen den zweiten Abschnitt 20. Vorzugsweise liegt die Außenseite 20a des zweiten Abschnitts 20 an der Innenseite 10b des Formkörpers 11 an. Um einen stabilen und dauerhaften Verbund zu gewährleisten, sind der erste Abschnitt 10 und der zweite Abschnitt 20 stoffschlüssig miteinander verbunden.

In der genannten Ausgestaltung des zweiten Abschnitts 20 durch einen Kunststoff, wird dieser vorzugsweise auf den ersten Abschnitt 10 oder Formkörper 11 aufgetragen oder aufgespritzt. Dabei stellt der Formkörper 11 bzw. die Innenseite 10b des Formkörpers 11 eine Art Negativform dar. Der zweite Abschnitt 20 soll im Wesentlichen eine gleitende Bewegung eines Gelenkkopfs 32 in der Gelenkpfanne 31 ermöglichen.

Dagegen soll der Formkörper 11 im Wesentlichen die dynamischen Eigenschaften des Implantats 100 bereitstellen und eine stabile Verankerung mit dem umliegenden Gewebe 50 oder Knochen 50 ermöglichen. Die zum Knochen 50 oder Gewebe 50 hin orientierte Außenseiten 10a der Implantate 100 ist, da der Formkörper 11 als gepresste Maschenware vorliegt, porös. Durch die Porösität des Formkörpers 11 wird das Einwachsen des Gewebes 50 in das Implantat 100 ermöglicht. Jedoch sollen hierbei die dynamischen Eigenschaften des Implantats 100 nicht wesentlich beeinträchtigt werden. Dies kann insbesondere erzielt werden, indem das Einwachsen des Gewebes 50 gezielt beeinflusst wird.

Dazu zeigt Figur 3.b eine Weiterbildung des Implanats 100 aus Figur 3.a. Dabei ist die Außenseite 10a, insbesondere zumindest in einem Oberflächenbereich, derart ausgebildet oder behandelt, dass ein Einwachsen des Gewebes 50 in die Außenseite 10a des Implantats 100, insbesondere in den genannten Oberflächenbereich, ermöglicht wird. Der Kern oder der Innenraum des Formkörpers 11 soll im Wesentlichen frei von dem Gewebe 50 bleiben, um die dynamischen Eigenschaften des Implantats 100 zu erhalten.

Dies wird zum Beispiel dadurch erzielt, dass die Drähte 12, die in der Außenseite 10a des Formkörpers 11 liegen, eine andere Oberfläche besitzen als die Drähte 12, die im Kern des Formkörpers 11 liegen. Figur 3.b illustriert hierzu schematisch das Implantat 100 aus Figur 3.a mit einer veränderten Außenseite 10a. Eine Möglichkeit stellt ein Draht 12 mit einer Beschichtung 14 dar.

Zum Beispiel ermöglicht Titan oder eine Titanlegierung ein Einwachsen von Gewebe 50 in einen porösen Körper. Edelstahl oder manche Kunststoffe dagegen verhindern oder behindern ein Einwachsen von Gewebe 50. Somit sind zwei Kombination zum Beispiel Titan/Edelstahl und Titan/Kunststoff. In einer Ausführungsform der Erfindung erfolgt das Ausbilden der Beschichtung 14 am bereits gebildeten Formkörper 11.

Vorzugsweise besitzt der Formkörper 11 hierbei bereits sein finales Aussehen. Im Detail wird die Außenseite 10a, vorzugsweise bis zu einer gewünschten Tiefe, präpariert.

In einer ersten Ausführungsform wird der Draht 12 des Formkörpers 11 aus einem Material, wie zum Beispiel Edelstahl, welches ein Einwachsen von Gewebe 50 unterdrückt, gebildet. Durch ein Beschichten des Drahtabschnitts, welcher die Außenseite 10a des Implantats 100 bildet, mit einem Material, wie zum Beispiel Titan oder eine Titanlegierung, kann ein Einwachsen des Gewebes 50 in die Außenseite 10a des Implantats 100 ermöglicht werden. Das Beschichten kann aus einem gasförmigen Zustand, zum Beispiel durch Aufdampfen, aus einem flüssigen oder pastenförmigen Zustand, zum Beispiel durch Spritzlackieren, und/oder durch elektrochemische Abscheidung aus Lösungen, zum Beispiel durch Galvanisieren, erfolgen. Das Galvanisieren ist insbesondere vorteilhaft, da über die Eintauchtiefe und/oder die Eintauchzeit des Implantats 100 bzw. der Außenseite 10a des Implantats 100 in eine Lösung die Tiefe und/oder die Dicke der Beschichtung 14 in dem Implantat gezielt eingestellt werden kann.

In einer zweiten Ausführungsform wird der Draht 12 des Formkörpers 11 aus einem Material, wie zum Beispiel Titan oder eine Titanlegierung gebildet, welches ein Einwachsen des Gewebes 50 ermöglicht. Der Draht 12 selbst ist dann, insbesondere bereits vor dem Verstricken, mit einem Überzug oder einer Beschichtung 14 aus einem Material, wie zum Beispiel Edelstahl oder einem Kunststoff, versehen, welches ein Einwachsen des Gewebes 50 unterdrückt. Durch ein Ablösen des Überzugs oder der Beschichtung 14 in dem Abschnitt, welcher die Außenseite 10a des Implantats 100 bildet, kann ein Einwachsen des Gewebes 50 in die Außenseite 10a des Implantats 100 ermöglicht werden. Das Ablösen kann zum Beispiel durch ein Ätzen erfolgen. Das Ätzen ist insbesondere vorteilhaft, da über die Eintauchtiefe und/oder Eintauchzeit des Implantats 100 bzw. der Außenseite 10a des Implantats 100 in eine ätzende Lösung oder Flüssigkeit die Tiefe des freigelegten Bereichs 14, hier des Titans oder der freigelegten Titanlegierung, in dem Formkörper 11 gezielt eingestellt werden kann.

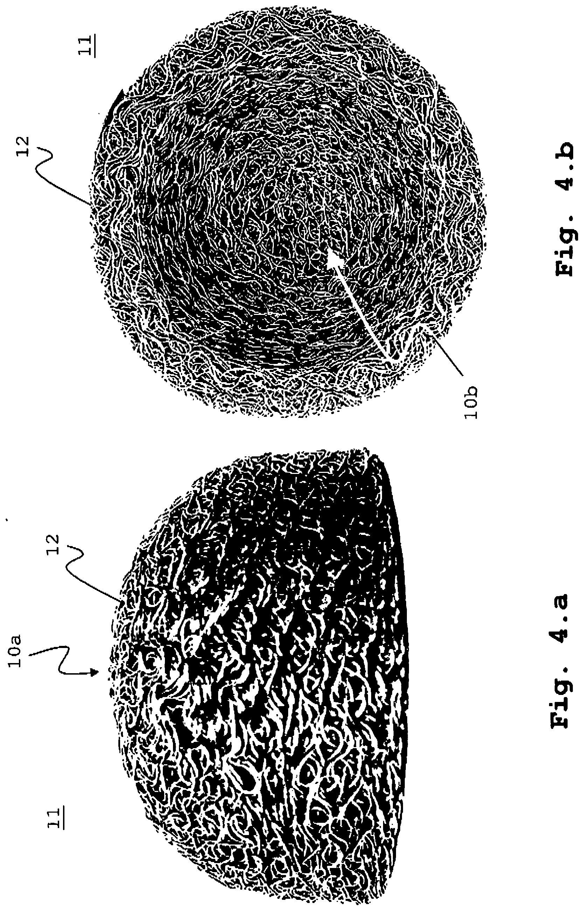

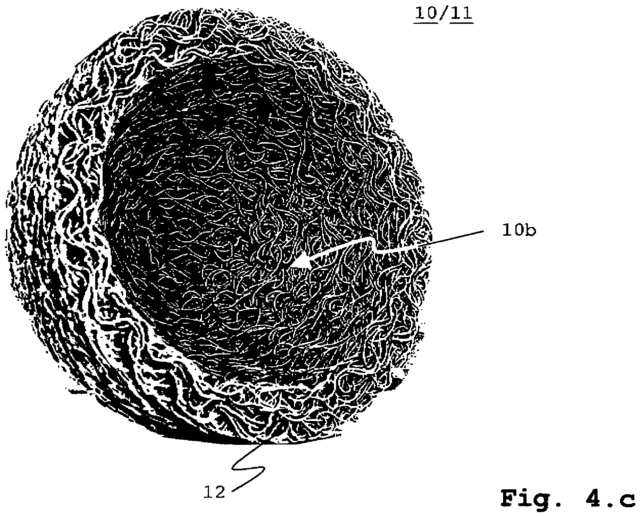

Die Figuren 4.a bis 4.c zeigen jeweils eine detaillierte Darstellung eines Formkörpers 11 in einer Seitenansicht, einer Unteransicht und einer perspektivischen Ansicht. Zu erkennen sind die Drähte 12 des Formkörpers 11, die in einer bevorzugten Ausgestaltung der Erfindung zunächst als eine rundgestrickte Maschenware bereitgestellt werden. In einem nächsten Schritt wird die Maschenware gefaltet und/oder gerollt. Dabei ist das Falten und/oder Rollen schon an die Form und/oder die Dichte der Maschenware angepasst, die der zu bildende Formkörper 11 besitzen soll. In einem nachfolgenden Schritt wird der Formkörper 11 durch ein Pressen der, vorzugsweise zunächst gefaltet und dann aufgerollten, Maschenware gebildet.

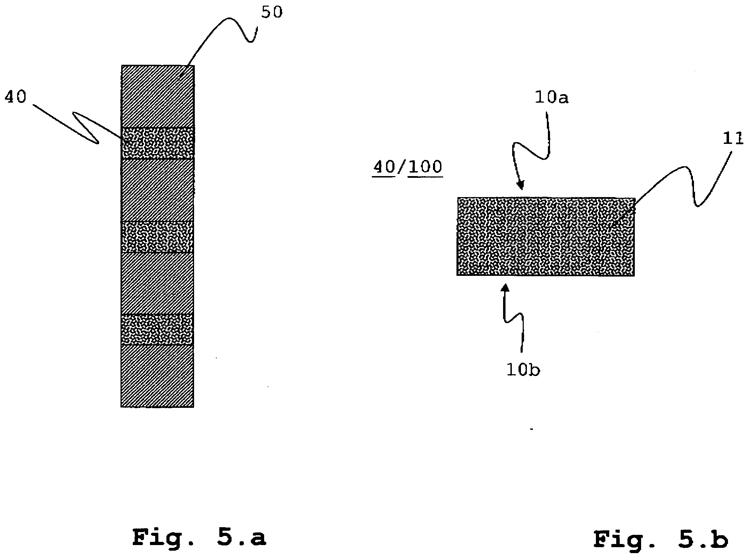

Die Figuren 5.a und 5.b illustrieren einen Ausschnitt einer Wirbelsäule und eines Implantats 100 für eine Bandscheibe 40. Das Implantat 100 liegt über seine Oberseite 10a und seine Unterseite 10b an den benachbarten Wirbeln 50 an. Vorzugsweise wird das Implantat 100, insbesondere ausschließlich, durch den Formkörper 11 bereitgestellt.

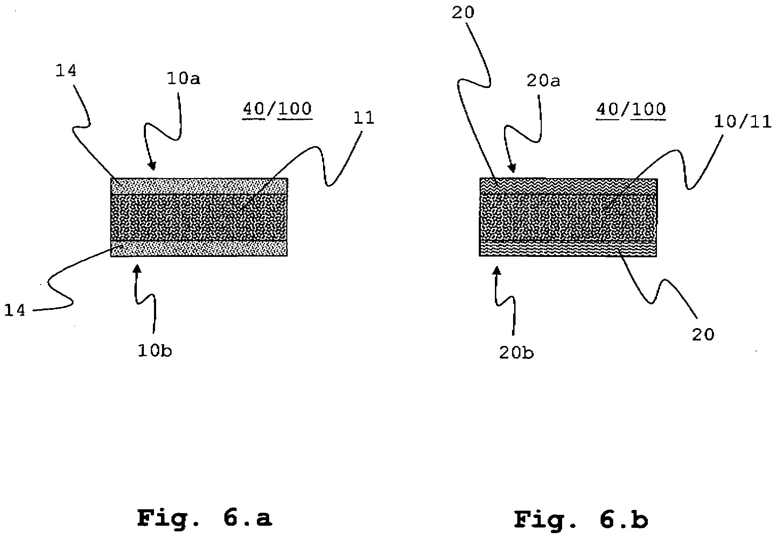

Um ein definiertes Einwachsen des Gewebes 50 oder des Knochens 50 zu ermöglichen, ist vorliegend sowohl die Oberseite 10a als auch die Unterseite 10b, vorzugsweise bis zu einer gewünschten Tiefe oder in einem Oberflächenbereich, des Implantats entsprechend ausgebildet. Figur 6.a zeigt hierzu schematisch einen Querschnitt eines erfindungsgemäß ausgebildeten Implantats 100 für eine Bandscheibe 40. Das Bezugszeichen 14 illustriert eine Beschichtung oder einen freigelegten Bereich. Für Details zu der Ausbildung der Oberseite 10a und der Unterseite 10b wird auf die Ausführungen zu Figur 3.b verwiesen, da diese entsprechend sind.

Figur 6.b zeigt den Querschnitt einer weiteren Ausführung eines Implantats 100 für eine Bandscheibe. Hierbei ist auf der Oberseite 10a und der Unterseite 10b des Formkörpers 11, hier als erster Abschnitt 10, jeweils ein zweiter, insbesondere nichtmetallischer, Abschnitt 20 angeordnet. Die beiden zweiten Abschnitte 20 stellen die Schnittstelle oder den Übergangsbereich zum benachbarten Gewebe 50 oder Knochen 50 dar. Der Formkörper 11 selbst ist sozusagen als ein elastischer Kern zwischen den beiden zweiten Körpern 20 angeordnet. Dadurch kann im Wesentlichen ein Einwachsen von Gewebe 50 oder Knochen 50 in den Formkörper 11 vermieden und dessen dynamische Eigenschaften erhalten werden. Die beiden zweiten Körper 20 können zum Beispiel auch eine poröse Struktur, insbesondere in ihrer Oberseite 20a und Unterseite 20b, besitzen, um ein Einwachsen des Gewebes 50 und somit eine feste Verankerung zu ermöglichen oder zu fördern. Vorzugsweise werden die beiden zweiten Körper 20 durch Kunststoff bereitgestellt und werden jeweils durch ein Aufspritzen auf den Formkörper 11 ausgebildet.

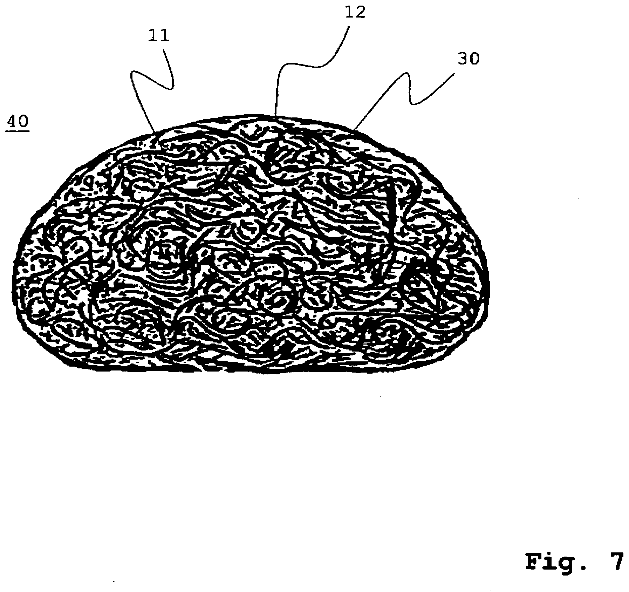

Abschließend zeigt die Figur 7 eine Detailansicht eines erfindungsgemäßen Implantats 100 für eine Bandscheibe 40 in einer Aufsicht. Zu erkennen sind wiederum die Drähte 12 und die Porosität des Formkörpers 11. Für weitere Details sei auf die Ausführungen zu den Figuren 4.a bis 4.c verwiesen.

Es ist dem Fachmann ersichtlich, dass die beschriebenen Ausführungsformen beispielhaft zu verstehen sind. Die Erfindung ist nicht auf diese beschränkt, sondern kann in vielfältiger Weise variiert werden kann, ohne den Geist der Erfindung zu verlassen. Merkmale einzelner Ausführungsformen und die im allgemeinen Teil der Beschreibung genannten Merkmale können jeweils untereinander als auch miteinander kombiniert werden.

### Bezugszeichenliste

- 10: Erster Abschnitt oder Körper
- 10a: Außenseite oder Oberseite des ersten Abschnitts oder des Formkörpers
- 10b: Innenseite oder Unterseite des ersten Abschnitts oder des Formkörpers
- 11: Formkörper
- 12: Draht
- 13: Schale oder Halbkugelschale
- 14: Beschichtung oder Überzug oder freigelegter Bereich
- 20: Zweiter Abschnitt
- 20a: Außenseite oder Oberseite des zweiten Abschnitts
- 20b: Innenseite oder Unterseite des zweiten Abschnitts
- 30: Gelenk oder Hüftgelenk
- 31: Gelenkpfanne
- 31a: Außenseite der Gelenkpfanne
- 31b: Innenseite der Gelenkpfanne
- 31c: Innenraum der Gelenkpfanne
- 32: Gelenkkopf
- 33: Schaft
- 40: Bandscheibe
- 50: Knochen oder Gewebe
- 100: Implantat

## Patentansprüche

1. Implantat (100) für den chirurgischen Einsatz bei Menschen oder Wirbeltieren zum Ersatz, zum Teilersatz oder zum Verstärken eines Gelenks (30) (40) umfassend einen Verbund aus
- einem ersten als Schale (13) mit einer Innenseite (10b) und einer Außenseite (10a) ausgebildeten Abschnitt (10), der durch einen gepressten elastischen Formkörper (11) mit einer anfänglichen Federkonstante von 50 bis 3000N/mm aus einer aufgerollten oder gefalteten Maschenware aus Draht (12) gebildet ist, wobei der gepresste Formkörper (11) im Wesentlichen die dynamischen Eigenschaften des Implantats (100) bereitstellt, und
- einem als Schale mit einer Innenseite (20b) und einer Außenseite (20a) ausgebildeten zweiten Abschnitt (20), wobei der erste Abschnitt (10) abschnittsweise in den zweiten Abschnitt (20) eingebettet ist,
wobei der zweite Abschnitt (20) an einer Seite des Implantats (100) angeordnet ist, welche eine Gleitfläche für eine Bewegung in einem ein Gelenk (30) bildet.

2. Implantat nach vorstehendem Anspruch 1 **dadurch gekennzeichnet, dass**
der zweite Abschnitt (20) ein nichtmetallisches Material ist und/oder dass der zweite Abschnitt (20) wenigstens ein Material aus einer Gruppe bestehend aus Keramik und Kunststoff aufweist.

3. Implantat nach einem der vorstehenden Ansprüche **dadurch gekennzeichnet, dass**
das Material zur Ausbildung des zweiten Abschnitts (20) auf den ersten Anschnitt (10) aufgespritzt ist und/oder dass das Material zur Ausbildung des zweiten Abschnitts (20) auf den ersten Abschnitt (10) aufgesintert ist.

4. Implantat nach einem der vorstehenden Ansprüche **dadurch gekennzeichnet, dass**
der erste Abschnitt (10) zur Ausbildung einer Gelenkpfanne (31) ausgebildet ist und dass der zweite Abschnitt (20) an einer Innenseite (10b) des ersten Abschnitts (10) angeordnet ist.

5. Implantat nach einem der vorstehenden Ansprüche, **dadurch gekennzeichnet, dass** der erste Abschnitt (10) an einer Seite des Implantats (100) angeordnet ist, die im eingebauten Zustand des Implantats (100) an das umliegende Gewebe grenzt, an dem die Befestigung erfolgen soll.

6. Implantat (100) nach einem der vorstehenden Ansprüche, weiter umfassend
einen gepressten Formkörper (11), der durch einen Draht (12) aus einem ersten Material gebildet ist, wobei der Draht (12) des Formkörpers (11) abschnittsweise eine Beschichtung (14) aus einem zweiten Material aufweist.

7. Implantat nach einem der vorstehenden Ansprüche **dadurch gekennzeichnet, dass**
der Formkörper (11) eine Beschichtung (14) an wenigstens einer seiner Seiten (10a, 10b), welche, im eingesetzten Zustand des Implantats (100) an ein umgebendes Gewebe (50) grenzt, aufweist, so dass ein Einwachsen des Gewebes (50) in einen Oberflächenbereich des Formkörpers (11) ermöglicht wird und/oder dass der Formkörper (11) keine Beschichtung (14) an wenigstens einer seiner Seiten (10a, 10b), welche, im eingesetzten Zustand des Implantats (100) an ein umgebendes Gewebe (50) grenzt, aufweist, so dass ein Einwachsen des Gewebes (50) in einen Oberflächenbereich des Formkörpers (11) ermöglicht wird.

8. Verfahren zur Herstellung eines Implantats (100) nach einem der vorstehenden Ansprüche, umfassend die Schritte:
- Bereitstellen einer Maschenware aus Draht (12),
- Falten und/oder Aufrollen der Maschenware,
- Pressen der Maschenware zu einem Formkörper (11) zum Ausbilden eines ersten Abschnitts (10),
- Bereitstellen eines zweiten Abschnitts (12) und
- Verbinden von dem ersten Abschnitts (10) und dem zweiten Abschnitt (20), wobei der erste Abschnitt (10) teilweise in dem zweiten Abschnitt (20) eingebettet wird.

9. Verfahren nach vorstehendem Anspruch **dadurch gekennzeichnet, dass**
der zweite Abschnitt (20) auf dem ersten Abschnitt (10) ausgebildet wird.

10. Verfahren nach einem der vorstehenden Ansprüche **dadurch gekennzeichnet, dass**
der zweite Abschnitt (20) auf dem ersten Abschnitt (10) aufgespritzt wird.

11. Verfahren nach einem der vorstehenden Ansprüche, weiter umfassend den Schritt:
- Ausbilden einer zumindest teilweisen Beschichtung auf dem Draht (12) mit einem zweiten Material, wobei das erste Material oder das zweite Material durch ein Material bereitgestellt wird, welches, im eingesetzten Zustand des Implantats (100), an einem Grenzbereich zu einem umgebenden Gewebe (50), abschnittsweise ein Einwachsen des Gewebes (50) in einen Oberflächenbereich (10a, 10b) des Formkörpers (11) ermöglicht.

12. Verfahren nach dem vorstehendem Anspruch **dadurch gekennzeichnet, dass**
zumindest eine Seite (10a, 10b) des Formkörpers (11) nach dem Pressen beschichtet wird, so dass, im eingebauten Zustand des Implantats (100), durch die ausgebildete Beschichtung (14) ein Verbund mit einem angrenzenden Gewebe (50), vorzugsweise durch ein Einwachsen des Gewebes (50) in den Formkörper (11), ermöglicht wird.

13. Verfahren nach einem der vorstehenden Ansprüche **dadurch gekennzeichnet, dass**
nach dem Pressen an zumindest einer Seite des Formkörpers (11) die Beschichtung (14) entfernt wird, so dass, im eingebauten Zustand des Implantats (100), durch die freigelegte Oberfläche des Drahts (12) ein Verbund mit einem angrenzenden Gewebe (50) durch ein Einwachsen des Gewebes (50) in den Formkörper (11), ermöglicht wird.

## Claims

1. An implant (100) for surgical use in human beings or vertebral animals for replacing, partially replacing, or strengthening a joint (30) (40), comprising a composite which comprises
- a first portion (10) with the shape of a shell (13) having an inner side (10b) and an outer side (10a), which is defined by an elastic pressed shaped body (11) having an initial spring constant from 50 to 3000 N/mm and made from a rolled-up or folded mesh of wire (12), wherein the pressed shaped body (11) substantially provides the dynamic properties of the implant (100); and
- a second portion (20) with the shape of a shell (23) having an inner side (20b) and an outer side (20a); wherein the first portion (10) is partially embedded in the second portion (20);
wherein the second portion (20) is arranged at a side of the implant (100) which defines a sliding surface for a movement in a joint (30).

2. The implant according to the preceding claim 1, **characterized in that** the second portion (20) is made of a non-metallic material and/or that the second portion (20) comprises at least one material from a group consisting of ceramics and plastics.

3. The implant according to any one of the preceding claims, **characterized in that** the material for forming the second portion (20) is sprayed onto the first portion (10); and/or that the material for forming the second portion (20) is sintered onto the first portion (10).

4. The implant according to any one of the preceding claims, **characterized in that** the first portion (10) is shaped so as to form a joint socket (31), and that the second portion (20) is arranged on an inner side (10b) of the first portion (10).

5. The implant according to any one of the preceding claims, wherein the first portion (10) is arranged at a side of the implant (100) which in the incorporated state of the implant (100) adjoins the surrounding tissue to which it is intended to be secured.

6. The implant (100) according to any one of the preceding claims, further comprising a pressed shaped body (11) which is formed from a wire (12) made of a first material, wherein the wire (12) of the shaped body (11) has a coating (14) of a second material on sections thereof.

7. The implant according to any one of the preceding claims, **characterized in that** the shaped body (11) has a coating (14) on at least one of its sides (10a, 10b) which in the incorporated state of the implant (100) adjoins a surrounding tissue (50), so as to allow ingrowth of the tissue (50) into a surface region of the shaped body (11); and/or that the shaped body (11) has no coating (14) on at least one of its sides (10a, 10b) which in the incorporated state of the implant (100) adjoins a surrounding tissue (50), so as to allow ingrowth of the tissue (50) into a surface region of the shaped body (11).

8. A method for producing an implant (100) according to any one of the preceding claims, comprising the steps of:
- providing a mesh of wire (12);
- folding and/or rolling up the mesh;
- pressing the mesh into a shaped body (11) to form a first portion (10);
- providing a second portion (12); and
- connecting the first portion (10) to the second portion (20) thereby partially embedding the first portion (10) into the second portion (20).

9. The method according to the preceding claim, **characterized in that** the second portion (20) is formed on the first portion (10).

10. The method according to any one of the preceding claims, **characterized in that** the second portion (20) is sprayed onto the first portion (10).

11. The method according to any one of the preceding claims, further comprising the step of:
- forming an at least partial coating on the wire (12) with a second material, wherein the first material or the second material is provided by a material which in the incorporated state of the implant (100) allows tissue (50) to grow into a surface region (10a, 10b) of the shaped body (11) in an interface region to the surrounding tissue (50).

12. The method according to the preceding claim, **characterized in that** at least one side (10a, 10b) of the shaped body (11) is coated after the pressing, so that in the incorporated state of the implant (100) the so formed coating (14) allows for a bond with the surrounding tissue (50), preferably through ingrowth of the tissue (50) into the shaped body (11).

13. The method according to any one of the preceding claims, **characterized in that** after the pressing, the coating (14) is removed on at least one side of the shaped body (11) so that in the incorporated state of the implant (100) the exposed surface of the wire (12) allows for a bond with the surrounding tissue (50), preferably through ingrowth of the tissue (50) into the shaped body (11).

## Revendications

1. Implant (100) pour une utilisation chirurgicale chez des humains ou des animaux vertébrés destiné à remplacer, à remplacer en partie ou à renforcer une articulation (30) (40), comprenant un composite fait :
- d'une première partie (10) qui est réalisée sous la forme d'une coque (13) pourvue d'une face intérieure (10b) et d'une face extérieure (10a) et qui est formée par un corps moulé (11) élastique pressé présentant une constante de rappel initiale de 50 à 3 000 N/mm et composé d'un tricot de fil métallique (12) enroulé ou plié, le corps moulé (11) pressé assurant sensiblement les propriétés dynamiques de l'implant (100), et
- d'une deuxième partie (20) réalisée sous la forme d'une coque pourvue d'une face intérieure (20b) et d'une face extérieure (20a),
la première partie (10) étant encastrée sur certaines parties dans la deuxième partie (20),
la deuxième partie (20) étant agencée sur une face de l'implant (100) qui forme une surface de glissement pour un mouvement dans une articulation (30).

2. Implant selon la revendication 1, **caractérisé en ce que** la deuxième partie (20) est un matériau non métallique et/ou **en ce que** la deuxième partie (20) comprend au moins un matériau choisi dans un groupe constitué de la céramique et de la matière plastique.

3. Implant selon l'une quelconque des revendications précédentes, **caractérisé en ce que** le matériau destiné à constituer la deuxième partie (20) est injecté sur la première partie (10) et/ou **en ce que** le matériau destiné à constituer la deuxième partie (20) est fritté sur la première partie (10).

4. Implant selon l'une quelconque des revendications précédentes, **caractérisé en ce que** la première partie (10) est conçue pour constituer une cavité articulaire (31) et **en ce que** la deuxième partie (20) est agencée sur une face intérieure (10b) de la première partie (10).

5. Implant selon l'une quelconque des revendications précédentes, **caractérisé en ce que** la première partie (10) est agencée sur une face de l'implant (100) qui, une fois l'implant (100) installé, est adjacente au tissu environnant sur lequel la fixation doit s'effectuer.

6. Implant (100) selon l'une quelconque des revendications précédentes, comprenant en outre :
un corps moulé (11) pressé, qui est formé par un fil métallique (12) fait d'un premier matériau, le fil métallique (12) du corps moulé (11) présentant sur certaines parties un revêtement (14) fait d'un deuxième matériau.

7. Implant selon l'une quelconque des revendications précédentes, **caractérisé en ce que** le corps moulé (11) présente un revêtement (14) sur au moins une de ses faces (10a, 10b), laquelle, une fois l'implant (100) inséré, est adjacente à un tissu (50) environnant, de sorte qu'un ancrage du tissu (50) dans une zone superficielle du corps moulé (11) est permis et/ou **en ce que** le corps moulé (11) ne présente aucun revêtement (14) sur au moins une de ses faces (10a, 10b), laquelle, une fois l'implant (100) inséré, est adjacente à un tissu (50) environnant, de sorte qu'un ancrage du tissu (50) dans une zone superficielle du corps moulé (11) est permis.

8. Procédé de fabrication d'un implant (100) selon l'une quelconque des revendications précédentes, comprenant les étapes consistant à :
- fournir un tricot composé de fil métallique (12),
- plier et/ou enrouler le tricot,
- presser le tricot de manière à obtenir un corps moulé (11) afin de constituer une première partie (10),
- fournir une deuxième partie (12), et
- relier la première partie (10) et la deuxième partie (20), la première partie (10) étant encastrée partiellement dans la deuxième partie (20).

9. Procédé selon l'une quelconque des revendications précédentes, **caractérisé en ce que** la deuxième partie (20) est formée sur la première partie (10).

10. Procédé selon l'une quelconque des revendications précédentes, **caractérisé en ce que** la deuxième partie (20) est injectée sur la première partie (10).

11. Procédé selon l'une quelconque des revendications précédentes, comprenant en outre l'étape consistant à :
- constituer un revêtement au moins partiel sur le fil métallique (12) au moyen d'un deuxième matériau, le premier matériau ou le deuxième matériau étant fourni par un matériau qui, une fois l'implant (100) inséré, au niveau d'une zone limite par rapport à un tissu (50) environnant, permet sur certaines parties un ancrage du tissu (50) dans une zone superficielle (10a, 10b) du corps moulé (11).

12. Procédé selon l'une quelconque des revendications précédentes, **caractérisé en ce qu'**au moins une face (10a, 10b) du corps moulé (11) est revêtue après le pressage, de sorte que, une fois l'implant (100) installé, du fait du revêtement (14) constitué, une liaison à un tissu (50) adjacent, de préférence à la suite d'un ancrage du tissu (50) dans le corps moulé (11), est permise.

13. Procédé selon l'une quelconque des revendications précédentes, **caractérisé en ce que**, après le pressage sur au moins une face du corps moulé (11), le revêtement (14) est éliminé, de sorte que, une fois l'implant (100) installé, du fait de la surface découverte du fil métallique (12), une liaison à un tissu (50) adjacent à la suite d'un ancrage du tissu (50) dans le corps moulé (11), est permise.
